# EUROPÄISCHE PATENTANMELDUNG

(11) **EP 1 355 158 A1**
(43) Veröffentlichungstag der Anmeldung: **22.10.2003**
(21) Anmeldenummer: 02008840.7
(22) Anmeldetag: 19.04.2002
(51) Int. Cl.: G01N 33/68

(54) **Verfahren zur Diagnose von Entzündungserkrankungen und Infektionen unter Bestimmung des Phosphoproteins LASP-1 als Inflammationsmarker**

(71) Anmelder: B.R.A.H.M.S Aktiengesellschaft, 16761 Hennigsdorf (DE)
(72) Erfinder: Bergmann, Andreas, Dr., 12351 Berlin (DE); Fischer-Schulz, Christina, 13507 Berlin (DE)
(74) Vertreter: Andrae, Steffen, Dr.

(57) **Zusammenfassung**

Verfahren zur Früherkennung und Erkennung, für die Verlaufsprognose und die Beurteilung des Schweregrads und zur therapiebegleitenden Verlaufsbeurteilung von Entzündungserkrankungen und Infektionen, insbesondere von Sepsis und sepsisähnlichen systemischen Infektionen sowie von Entzündungserkrankungen, die im Gehirn lokalisiert sind, einschließlich der Alzheimer Krankheit, oder die auf das Gehirn übergreifen, bei dem man die Anwesenheit und/oder Menge einer Expressionsform des Phosphoproteins LASP-1 in einer biologischen Flüssigkeit auf Blut- oder Liquorbasis oder in einer Gewebeprobe eines Patienten bestimmt und aus der Anwesenheit und/oder Menge von LASP-1 Schlüsse hinsichtlich des Vorliegens, des zu erwartenden Verlaufs, des Schweregrads oder des Erfolgs einer Therapie der Entzündungserkrankung oder der Infektion zieht.

## Beschreibung

Die vorliegende Erfindung betrifft neue Verwendungen des Proteins LASP-1 für die medizinischen Diagnostik von Entzündungserkrankungen und Infektionen, insbesondere von Sepsis und sepsisähnlichen systemischen Infektionen sowie von Entzündungserkrankungen, die im Gehirn lokalisiert sind oder die auf das Gehirn übergreifen. Sie beruht auf dem erstmaligen Nachweis stark erhöhter Konzentrationen des Proteins LASP-1 im Gehirngewebe von Primaten, bei denen experimentell durch Toxinverabreichunge eine Sepsis bzw. systemische Entzündung hervorgerufen wurde.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen. Als Erkrankung, die von typischen Symptomen einer Entzündung, und zwar des Gehirns, begleitet wird, ist auch die Alzheimer Krankheit anzusehen, die durch eine in der Regel im 5. bis 6. Lebensjahrzehnt auftretende, unaufhaltsam fortschreitende Großhirnrindenatrophie charakterisiert ist und aufgrund ihrer Häufigkeit eine große volkswirtschaftliche Bedeutung aufweist.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α, Interleukin-1) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß oder vermutet, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen oder Ausprägungen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund (gute Spezifität und Selektivität), ohne dass dessen Rolle in der komplexen Kaskade der am Entzündungsgeschehen beteiligten endogenen Substanzen bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Infektionsgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung werden Ergebnisse eines anderen, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procaicitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene entzündugs- und/oder sepsisspezifische Biomarker von peptidischer bzw. proteinischer Natur zu ermitteln, deren Auftreten für bestimmte Formen von Entzündungen und/oder Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Die vorliegende Erfindung beruht darauf, dass sich in Primaten und Menschen bei infektiös bedingten Entzündungen im cytoplasmatischen Gehirngewebe das stark überexprimierte Protein LASP-1 in einer löslichen Form nachweisen läßt, und zwar im Unterschied zu Gesunden bzw. Unbehandelten, bei denen es nicht oder nur in Konzentrationen an der analytischen Nachweisgrenze gefunden wird, und auch im Unterschied zu anderen Körpergeweben septischer Primaten-Versuchstiere, was dieses Protein für die Entzündungsdiagnostik und Infektions- bzw. Sepsisdiagnostik mit Gehirnbeteiliung geeignet macht.

Die Verwendungen in der Diagnostik, die sich aufgrund des erstmals nachgewiesenen gehirnspezifischen Auftretens von LASP-1 bei der experimentellen Simulation von Entzündungen bzw. Sepsis ergeben, werden nachfolgend noch näher beschrieben.

Die Ansprüche 1 bis 9 definieren die sich ergebenden neuen Verfahren gemäß der vorliegenden Erfindung, und bevorzugter Ausführungsformen davon, näher.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Gehirngewebe der behandelten Tiere als eines der nur bei den behandelten Tieren identifizierbaren Produkte das Protein LASP-1 gefunden werden konnte, das außerdem nur im Gehirngewebe in erheblich erhöhter Konzentration beobachtet wurde. Zu seiner Identifizierung wurde ein neuer Proteinspot, der nur im Gehirngewebe der behandelten Tiere gefunden wurde und gemäß Gelektrophorese ein Molekulargewicht von etwa 36 ± 3 kDA und einen isoelektrischen Punkt von ca. 6,6-7,0 aufwies, ung in Bruchstücke zerlegt, die auf an sich bekannte Weise massenspektrometrisch analysiert wurden. Durch Vergleich mit dem bekannten Massenspektrum des trypsinbehandeltem Proteins LASP-1 wurde das Protein des isolierten Spots als LASP-1 identifiziert.

Die Identifizierung des in dem Proteinspot gefundenen Produkts wird im expermientellen Teil näher beschrieben, wobei die beschriebene Identifizierung nach anerkannten Interpretationsgrundsätzen als sichere Identifizierung des Produkts als LASP-1 anzusehen ist.

Das rechnerisch ermittelte Molekulargewicht der Peptidkette von LASP-1 (vgl. SEQ ID NO:1; 261 Aminosäuren) beträgt 29 717 Dalton. LASP-1 liegt jedoch physiologisch in post-translational prozessierter Form (glykosyliert und phosphoryliert) vor. So wurde das später als LASP-1 bezeichnete Produkt ursprünglich als ein 40 kDA CAMP-abhängiges Phosphoprotein ("pp40") in Magenwandzellen identifiziert (vgl. C.S. Chew et al., Journal of Cell Science 113, 2035-2045 (2000); C.S. Chew et al., Am.J.Physiol. 275 (Cell Physiol. 44): C56-C67, (1998)).

Die Aminosäuresequenz des als LASP-1 bezeichneten Phosphoproteins wurde ursprünglich als die des putativen Expressionsprodukts der cDNA eines in humanen Brustkrebs-Zellinien überexprimierten Gens "MLN 50" aus dem Bereich q11-q21.3 des Chromosoms 17 ermittelt (C.Tomasetto at al., FEBS Letters 373 (1995): 245-249; C.Tomasetto et al., Genomics 28: 367-376 (1995); I.Bièche et al., Cancer Res. 56: 3886-3890 (1996)). Der Name LASP-1 geht darauf zurück, dass in diesem Protein erstmals eine sog. LIM-Domäne (eine aus verschiedenen Proteinen bekannte Zn-bindende Domäne; vgl. A.Hammarström et al., Biochemistry 1996, 35, 12723-12732) mit einer aus der rezeptorunabhängigen sog. src-Kinase, einer Tyrosin-Kinase, bekannten Domäne (SH3) kombiniert ist (LIM and SH3 Protein); vgl. auch Brian K. Kay et al., FASEB J. 14, 231-241 (2000).

LASP-1-mRNA ist ubiquitär in normalen Zellen nachweisbar (z.B. Prostata, Leber, Muskel, Gehirn, verschiedenen ZellTypen) und wird in einem gewissen prozentualen Anteil von Brustkrebsfällen überexprimiert. Es wurde festgestellt, dass LASP-1 ein Actin-bindendes Protein ist, für das angenommen wird, dass es zu den Cytoskelett-assoziierten Proteinen zu zählen ist und für die Zellform und -beweglichkeit sowie möglicherweise die Signalübertragung von Bedeutung ist. Es ist auch in Nervenenden nachweisbar. LASP-1 wird durch Detergentien nur schwer solubilisiert. In vitro hat sich LASP-1 als Substrat erwiesen, dass sich hervorragend mit der cAMP-abhängigen Serin/Threonin-Kinase PKA phosphorylieren läßt. LASP-1 weist in unterschiedlichen Epithel-Zelltypen unterschiedliche Expressionsmuster auf (vgl. C.S. Chew et al., Journal of Cell Science 113, 2035-2045 (2000); C.S. Chew et al., Am.J.Physiol. 275 (Cell Physiol. 44): C56-C67, (1998)).

Das Auftreten von LASP-1 in überexprimierter Form im Gehirngewebe bei Sepsis ist von hohem wissenschaftlichen, diagnostischen und möglicherweise auch therapeutischen Interesse. Die Tatsache, dass LASP-1 ein Kinase-Substrat und Phosphoprotein ist, verleiht ihm eine gewisse Ähnlichkeit mit den sogenannen tau-Proteinen, die bei der Alzheimer Krankheit eine Rolle insbesondere für diagnostische Zwecke spielen (vgl. z.B. G.A.Jicha et al., Journal of Neurochemistry,69., 2087-2095 (1997) und die darin zitierte Literatur; sowie EP 673 418; EP 737 208; EP 772 634; EP 610 330; und EP 618 968).

Es ist nunmehr gut bekannt, dass die Alzheimer Krankheit als gehirnspezifischer Entzündungsprozess angesehen werden kann, ohne dass damit eine Aussage bezüglich Krankheitsursache oder Krankheitsfolge gemacht werden soll (Lih-Fen Lue et al., GLIA 35:72-79, 2001; Michael Hüll et al., DDT Vol.4, No.6: 275-282; (June 1999); F.Licastro et al., Journal of Neuroimmunologie 103 (2000), 97-102; Neuroinflammation Working Group: Haruhiko Akiyama et al., Neurobiology of Aging 21 (2000) 383-421; Michael Hüll et al., Exp.Opin.Invest.Drugs (2000) 9(4) :671-683; M.Hüll et al., Eur Arch Psychiatry Clin Neurosci (1996) 246:124-128; J.Rhodin et al., Annals New York Academy of Sciences, 199x, 345-352). Die Tatsache, dass das Phosphoprotein LASP-1 unter experimentellen Bedingungen, die eine Sepsis bzw. ein systemisches Entzündungsgeschehen simulieren, gehirnspezifisch in überexprimierter Form nachweisbar ist, macht es daher sehr wahrscheinlich, dass auch bei der Alzheimer Krankheit eine Überexpression von Lasp-1 bzw. einer bestimmten Expressionsform von LASP-1 beobachtebar ist und die Bestimmung von LASP-1 daher auch für die Alzheimer-Diagnostik Bedeutung erlangen wird.

Die bisherigen Publikationen zu LASP-1 deuten eine solche Möglichkeit in keiner Weise an. Es war aufgrund der bisher zugänglichen Befunde nicht zu erwarten, das die physiologischen Konzentrationen von LASP-1 im Gehirn als Folge einer Sepsis und/oder Entzündung signifikant und nachweisbar verändert sind und daher eine Bestimmung der LASP-1-Konzentrationen im Rahmen eines Sepsisgeschehens oder einer entzündlichen Gehirnerkrankung diagnostisch von Interesse sein könnte.

Der erfindungsgemäße Nachweis von vergleichsweise hohen LASP-1-Konzentrationen im Gehirngewebe von Primaten, bei denen durch Toxinverabreichung eine künstliche Sepsis ausgelöst wurde, bei gleichzeitiger Unmöglichkeit, LASP-1 in ansonsten völlig gleich behandelten Proben von Kontrolltieren, oder in anderen Gewebeproben der septischen Tiere, zu erkennen, ist hoch signifikant. Da das Auftreten nur bei den behandelten Tieren zu beobachten war, und zwar bereits relativ kurze Zeit nach der Sepsisauslösung durch Toxinverabreichung, ist es möglich, diese Tatsache zur Schaffung eines vielversprechenden diagnostischen Sepsis-, Infektions- und Entzündungs-Nachweisverfahrens durch Bestimmung von LASP-1 zu nutzen. Von besonderem Interesse ist außerdem eine möglich erscheinende Eignung von LASP-1 als diagnostischer Marker und Prognosemarker für die Alzheimer Krankheit.

Die Bestimmung von LASP-1 kann nach irgendeinem beliebigen geeigneten Nachweisverfahren erfolgen, wobei jedoch die Bestimmung in einer Körperflüssigkeit, einschließlich Liquor (Liquor cerebrospinalis), eines Patienten auf immundiagnostischem Wege (mittels eines Immunoassays) unter Verwendung geeigneter selektiver Antikörper unter praktischen Gesichtspunkten am vorteilhaftesten erscheint.

Aufgrund der Tatsache, dass bei einer experimentellen Sepsisauslösung erstmals das erhöhte Auftreten von LASP-1 im Gehirn nachgewiesen werden konnte, wird somit die Möglichkeit geschaffen, LASP-1 insbesondere für diagnostische Zwecke im Zusammenhang mit Entzündungen und Infektionen, die sich im Gehirn manifestieren, zu nutzen. Dafür können LASP-1 oder geeignete Teilpeptide davon ggf. auch nach Verfahren, die inzwischen zum Stand der Technik gehören, synthetisch oder gentechnologisch gezielt hergestellt werden. LASP-1-Teilpeptide, ggf. in markierter Form, können auch als Kalibratoren, Tracer und Kompetitoren für bestimmte Assayformate für immundiagnostische Bestimmungen benötigt und dafür wie erläutert hergestellt werden.

Ferner können LASP-1-Fragmente bzw. geeignete Teilsequenzen davon nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung von LASP-1 in Körperflüssigkeiten eines Patienten und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik und muss nicht besonders beschrieben werden. Ferner ist auch ausdrücklich die Antikörpererzeugung unter Anwendung von Techniken der direkten genetischen Immunisierung mit einer entsprechenden DNA zu erwähnen. Es liegt somit im Rahmen der vorliegenden Erfindung, zur Immunisierung z.B. eine cDNA von LASP-1 oder LASP-1-Fragmenten zu verwenden, da es sich in der Vergangenheit gezeigt hat, dass bei der Anwendung derartiger Immunisierungstechniken das Spektrum der gewinnbaren Antikörper erweitert werden kann. Es können aber auch bereits bekannte Antikörper gegen LASP-1 verwendet werden (vgl. z.B. V.Schreiber et al., Molecular Medicine 4: 675-687, 1998; C.S. Chew et al., Am.J.Physiol. 275 (Cell Physiol. 44): C56-C67, (1998)).

Da im Rahmen der vorliegend beschriebenen Arbeiten im Gel des 2D-Elektrophorese ein LASP-1-Produkt mit einer molaren Massen von ca. **36 kDa** gefunden wurde, das eine für die beschriebene Bestimmung ausreichende Löslichkeit aufweist, ist zu vermuten, dass das gefundene LASP-1-Produkt eine bestimmte Art der Glykosylierung und/oder Phosphorylierung aufweist, die ihm die beobachtete Löslichkeit verleiht und aufgrund derer es sich von dem aus der Magenwand isolierten 40 kDa-Produkt signifikant unterscheidet. Es liegt daher auch im Rahmen der vorliegenden Erfindung, die Bestimmung von LASP-1 mit Hilfe eines spezifischen Assays durchzuführen, bei dem spezifische Glykosylierungs- und/oder Phosphorylierungsmuster erkannt werden. Die Herstellung von Antikörpern, insbesondere monoklonalen Antikörpern, die spezifische Glykosylierungen und/oder Phosphorylierungen erkennen, ist grundsätzlich bekannt und z.B. im Zusammenhang mit der Bestimmung bestimmter Phosphorylierungsformen der sogenannten tau-Proteine im Rahmen der Alzheimer-Diagnostik beschrieben.

Bei der immunologischen Bestimmung von LASP-1 kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851. Variationen der beschriebenen Techniken und/oder weitere Immunisierungstechniken kann der Fachmann einschlägigen Standardwerken und Veröffentlichungen entnehmen und sinngemäß anwenden.

LASP-1 oder ggf. LASP-1-Fragmente können aufgrund der vorliegenden Ergebnisse als spezifische Markerpeptide (Biomarker) zum diagnostischen Nachweis und zur Verlaufskontrolle von Entzündungen und Infektionen (insbesondere auch von systemischen Infektionen vom Sepsistyp und Entzündungen des Gehirns) dienen. Wie die Bestimmung von Procalcitonin kann dabei die Bestimmung von LASP-1 zur differentialdiagnostischen Früherkennung und zur Erkennung sowie für die Erstellung einer Verlaufsprognose, zur Beurteilung des Schweregrads und zur therapiebegleitenden Verlaufsbeurteilung von Entzündungen und Infektionen erfolgen, wobei man bei einem derartigen Verfahren in einer Probe einer biologischen Flüssigkeit, einschließlich des sog. Liquors, oder eines Gewebes eines Patienten den Gehalt von LASP-1 bestimmt und aus der festgestellten Anwesenheit und/oder Menge von LASP-1 auf das Vorliegen einer Entzündung mit Gehirnbeteiligung oder einer Sepsis schließt und das erhaltene Ergebnis mit dem Schweregrad der Sepsis korreliert und ggf. die Behandlungsmöglichkeiten und/oder die Behandlungsaussichten abschätzt.

Besonders interessant erscheint eine Bestimmung von LASP-1 im Rahmen einer Multiparameter-Bestimmung, bei der gleichzeitig mindestens ein weiterer Entzündungs- oder Infektionsparameter bestimmt wird und bei der ein Messergebnis in Form eines Satzes von mindestens zwei Messgrößen gewonnen wird, eines Satzes von mindestens zwei Messgrößen gewonnen wird, der zur Feindiagnostik der Entzündung oder Infektion ausgewertet wird. Als derartige weitere Entzündungs- oder Infektionsparameter sind solche anzusehen, die aus der Gruppe der teilweise bekannten oder in den älteren bzw. parallelen Patentanmeldungen der Anmelderin offenbarten Parameter ausgewählt sind, die besteht aus Procalcitonin, CA 125, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT), der Carbamoylphosphat Synthetase 1 (CPS 1) und deren Fragmenten und dem C-reaktiven Protein (CRP) oder Fragmenten aller genannten Proteine. Es ist vorteilhaft, die Multiparameter-Bestimmung als Simultanbestimmung mittels einer Chiptechnologie-Messvorrichtung oder einer immunchromatographischen Messvorrichtung durchzufüren, bei der die Auswertung des mit der Messvorrichtung gewonnenen komplexen Messergebnisses mit Hilfe eines Computerprogramms erfolgt.

Nachfolgend wird die Auffindung und Identifizierung von LASP-1 in näheren Einzelheiten geschildert. Die Figuren zeigen:
- Fig. 1: Ansichten von 2D-Elektrophoresegelen, die einen Vergleich der Spotmuster der cytoplasmatischen Gehirnproteine eines gesunden Pavians (A) mit denen der Gehirnproteine eines Pavians 5h nach einer durch LPS-Verabreichung induzierten Sepsis (B) ermöglichen. Der Pfeil zeigt die Position des erfindungsgemäßen sepsisspezifischen Produkts (LASP-1) an, das in Darstellung (B) durch einen Kreis hervorgehoben ist;

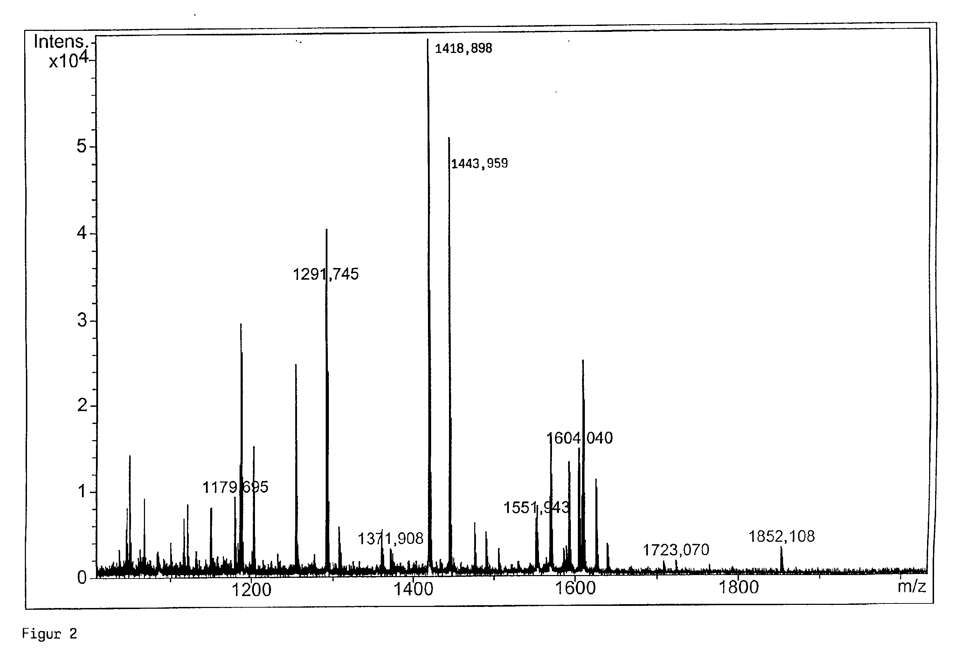

- Fig. 2: das Massenspektrum des aus dem Gel der 2D-Gelelektrophorese isolierten, trypsinverdauten Produkts.

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

### 2. Proteomanalyse unter Verwendung cytoplasmatischer Gehirnproteine von Pavianen.

Cytoplasmatische Gehrinzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Gehirnextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

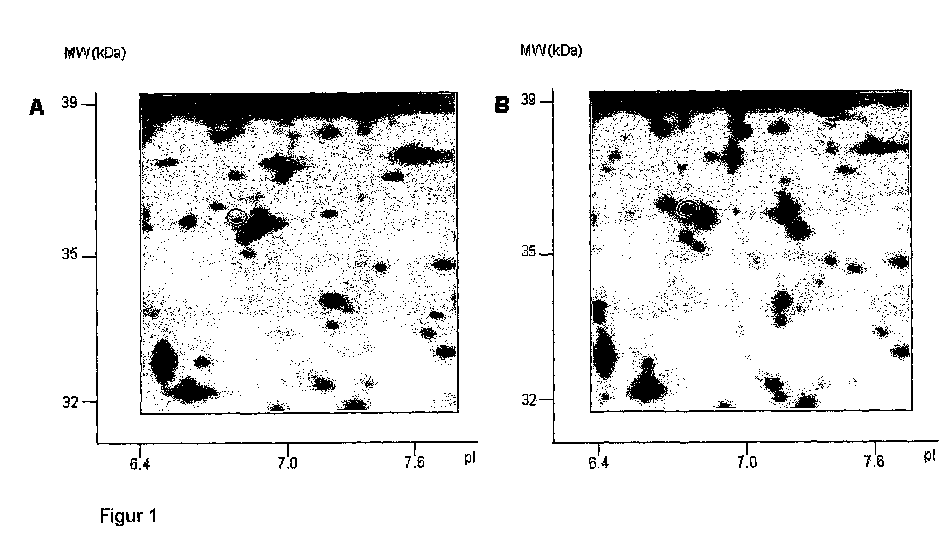

Zur Auswertung wurden die Proteinspotmuster der Proben behandelter Tieren mit den Proteinspotmustern verglichen, die aus Gehirngewebeproben unbehandelter Tiere resultierten. Ferner wurden die Proteinspotmuster aus dem Gehirngewebe behandelter Tiere auch mit denen anderer Gewebe derselben behandelten Tiere verglichen (die Ergebnisse sind nicht im Einzelnen gezeigt). Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) einem neuen Protein entspricht, dessen Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten, unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Die trypsinverdauten Proben wurden einer MALDI-TOF Massenspektrometrie unterzogen.

### 3. Identifizierung von LASP-1

Wie in den Figuren 1(A) und 1(B) gezeigt ist, findet sich in Gehirngewebeextrakten von Pavianen, denen eine LPS-Injektion verabreicht worden war, u.a. ein Spot eines Proteins, für das aufgrund der Gelektrophoresedaten im Vergleich mit Markersubstanzen mit bekanntem Molekulargewicht ein Molekulargewicht von ca. 36 ± 3 kDa abgeschätzt wurde, während aus der relativen Position des Proteins aus der ersten Dimension ein isoelektrischer Punkt von ca. 6,6-7,0 ermittelt wurde.

Bei der Massenanalyse des tryptischen Verdaus des neuen Proteinspots wurde für 21 Bruchstücke unter Rückgriff auf Datenbankinformationen der Versuch einer Zuordnung zu Peptiden vorgenommen. Im Ergebnis entsprachen zwölf der erhaltenen Zuordnungen Bruchstücken der Proteinkette des Phosphoproteins LASP-1 (SEQ ID NO:1). Die sog. "Sequence Coverage" betrug 38%, was nach den anerkannten fachüblichen Kriterien als eindeutige Identifizierung des in dem Spot enthaltenen Proteins als Proteinkette von LASP-1 anzusehen ist.

So konnten z.B. die folgenden Massen Bruchstücken der LASP-1-Proteinkette (SEQ ID NO.1) zugeordnet werden: 1202,714; 1254,615; 1291,745; 1360,813; 1418,898; 1443,959; 1489,826; 1551,943; 1604,040; 1608,960; und zwar war die Zuordnung gemäß MS-Fit 3.3.1. Protein Prospector 3.4.1., Database NCBInr.7.25.2002 wie folgt:

Die Masse 1202,714 (MH⁺) entspricht der Sequenz QQSELQSQVR (SEQ ID NO:2). Das theoretische Molekulargewicht dieses Fragments beträgt 1201,604. In der Aminosäuresequenz von LASP-1 findet sich diese Sequenz an den Positionen 76-95 wieder.

Die Masse 1254,615 entspricht der Sequenz ACFHCETCK (SEQ ID NO:3). Das theoretische Molekulargewicht dieses Fragments beträgt 1253,498. In der Aminosäuresequenz von LASP-1 findet sich diese Sequenz an den Positionen 28-36 wieder.

Die Masse 1291,745 entspricht der Sequenz KPYCNAHYPK (SEQ ID NO:4). Das theoretische Molekulargewicht dieses Fragments beträgt 1290.617. In der Aminosäuresequenz von LASP-1 findet sich diese Sequenz an den Positionen 50-59 wieder.

Die Masse 1360,813 entspricht der Sequenz VNCLDKFWHK (SEQ ID NO:5). Das theoretische Molekulargewicht dieses Fragments beträgt 1359,675. In der Aminosäuresequenz von LASP-1 findet sich diese Sequenz an den Positionen 18-27 wieder.

Die Masse 1418,898 entspricht der Sequenz GFSVVADTPELQR (SEQ ID NO:6). Das theoretische Molekulargewicht dieses Fragments beträgt 1417,719. In der Aminosäuresequenz von LASP-1 findet sich diese Sequenz an den Positionen 97-109 wieder.

Die Masse 1443,959 entspricht der Sequenz LKQQSELQSQVR (SEQ ID NO:7). Das theoretische Molekulargewicht dieses Fragments beträgt 1442,783. In der Aminosäuresequenz von LASP-1 findet sich diese Sequenz an den Positionen 74-85 wieder.

Die Masse 1489,826 entspricht der Sequenz MGPSGGEGMEPERR (SEQ ID NO:8). Das theoretische Molekulargewicht dieses Fragments beträgt 1488,644. In der Aminosäuresequenz von LASP-1 findet sich diese Sequenz an den Positionen 131-144 wieder.

Die Masse 1551,943 entspricht der Sequenz TGDTGMLPANYVEAI (SEQ ID NO:9). Das theoretische Molekulargewicht dieses Fragments beträgt 1550,728. In der Aminosäuresequenz von LASP-1 findet sich diese Sequenz an den Positionen 247-261 wieder.

Die Masse 1604,040 entspricht der Sequenz GKGFSVVADTPELQR (SEQ ID NO:10). Das theoretische Molekulargewicht dieses Fragments beträgt 1602,836. In der Aminosäuresequenz von LASP-1 findet sich diese Sequenz an den Positionen 95-109 wieder.

Die Masse 1608,960 entspricht der Sequenz QSFTMVADTPENLR (SEQ ID NO:11). Das theoretische Molekulargewicht dieses Fragments beträgt 1607,761. In der Aminosäuresequenz von LASP-1 findet sich diese Sequenz an den Positionen 60-73 wieder.

Eine solche Übereinstimmung gilt nach anerkannten Interpretationsgrundsätzen als sichere Identifizierung des Produkts aus dem Proteinspot als LASP-1. Das Molekulargewicht von unphosporyliertem LASP-1 (261 Aminosäuren; vgl. SEQ ID NO:1) beträgt 29 717 Dalton. Das nachgewiesene Protein muß daher in einer post-translational prozessierten Form (glykosyliert sowie ggf. phosphoryliert) vorgelegen haben.

Die erstmals festgestellten erhöhten LASP-1-Konzentrationen erlauben dessen Verwendung als neuer Biomarker für Sepsis und Entzündungen, insbesondere infektiöse Entzündungen.

## Patentansprüche

1. Verfahren zur Früherkennung und Erkennung, für die Verlaufsprognose und die Beurteilung des Schweregrads und zur therapiebegleitenden Verlaufsbeurteilung von Entzündungserkrankungen und Infektionen, insbesondere sepsisähnlichen systemischen Infektionen, **dadurch gekennzeichnet, dass** man die Anwesenheit und/oder Menge des Proteins LASP-1 in einer biologischen Flüssigkeit oder einer Gewebeprobe eines Patienten bestimmt und aus dessen Anwesenheit und/oder Menge Schlüsse hinsichtlich des Vorliegens, des zu erwartenden Verlaufs, des Schweregrads oder des Erfolgs einer Therapie der Entzündungserkrankung oder der Infektion zieht.

2. Verfahren nach Anspruch 1, **dadurch gekennzeichnet, dass** man eine posttranslational gebildete lösliche Form von LASP-1 bestimmt, und das LASP-1-Protein bei seiner Bestimmung ein- oder mehrfach phosphoryliert oder in nicht-phosphoryliert vorliegt.

3. Verfahren nach Anspruch 1 oder 2, **dadurch gekennzeichnet, dass** die biologische Flüssigkeit Blut, eine Blutfraktion oder Liquor cerebrospinalis ist.

4. Verfahren nach einem der Ansprüche 1 bis 3, **dadurch gekennzeichnet, dass** es ein immundiagnostisches Bestimmungsverfahren (Immunoassay) ist.

5. Verfahren nach einem der Ansprüche 1 bis 4, **dadurch gekennzeichnet, dass** die Entzündungserkrankung eine entzündliche Erkrankung des Gehirns, insbesondere die Alzheimer Krankheit, ist.

6. Verfahren nach einem der Ansprüche 1 bis 5, **dadurch gekennzeichnet, dass** es im Rahmen einer Multiparameter-Bestimmung durchgeführt wird, bei der gleichzeitig mindestens ein weiterer Entzündungs- oder Infektionsparameter bestimmt wird und bei der ein Messergebnis in Form eines Satzes von mindestens zwei Messgrößen gewonnen wird, der zur Feindiagnostik der Entzündung oder Infektion ausgewertet wird.

7. Verfahren nach Anspruch 6, **dadurch gekennzeichnet, dass** im Rahmen der Multiparameter-Bestimmung neben LASP-1 wenigstens ein weiterer Parameter bestimmt wird, der ausgewählt ist aus der Gruppe, die besteht aus Procalcitonin, CA 125, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT), der Carbamoylphosphat Synthetase 1 (CPS 1) und dem C-reaktiven Protein (CRP) oder Fragmenten davon.

8. Verfahren nach Anspruch 6 oder 7, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

9. Verfahren nach Anspruch 8, **dadurch gekennzeichnet, dass** die Auswertung des mit der Messvorrichtung gewonnenen komplexen Messergebnisses mit Hilfe eines Computerprogramms erfolgt.


SEQUENCE LISTING

<110> B.R.A.H.M.S Aktiengesellschaft

<120> Verfahren zur Diagnose von Entzündungserkrankungen und
      Infektionen unter Bestimmung des Phosphoproteins LASP-1
      als Inflammationsmarker

<130> 3677 AS

<140>
<141>

<160> 11

<170> PatentIn Ver. 2.1

<210> 1
<211> 261
<212> PRT
<213> Homo sapiens

<400> 1
Met Asn Pro Asn Cys Ala Arg Cys Gly Lys Ile Val Tyr Pro Thr Glu
  1               5                  10                  15

Lys Val Asn Cys Leu Asp Lys Phe Trp His Lys Ala Cys Phe His Cys
             20                  25                  30

Glu Thr Cys Lys Met Thr Leu Asn Met Lys Asn Tyr Lys Gly Tyr Glu
         35                  40                  45

Lys Lys Pro Tyr Cys Asn Ala His Tyr Pro Lys Gln Ser Phe Thr Met
     50                  55                  60

Val Ala Asp Thr Pro Glu Asn Leu Arg Leu Lys Gln Gln Ser Glu Leu
 65                  70                  75                  80

Gln Ser Gln Val Arg Tyr Lys Glu Glu Phe Glu Lys Asn Lys Gly Lys
                 85                  90                  95

Gly Phe Ser Val Val Ala Asp Thr Pro Glu Leu Gln Arg Ile Lys Lys
            100                 105                 110

Thr Gln Asp Gln Ile Ser Asn Ile Lys Tyr His Glu Glu Phe Glu Lys
        115                 120                 125

Ser Arg Met Gly Pro Ser Gly Gly Glu Gly Met Glu Pro Glu Arg Arg
    130                 135                 140

Asp Ser Gln Asp Gly Ser Ser Tyr Arg Arg Pro Leu Glu Gln Gln Gln
145                 150                 155                 160

Pro His His Ile Pro Thr Ser Ala Pro Val Tyr Gln Gln Pro Gln Gln
                165                 170                 175

Gln Pro Val Ala Gln Ser Tyr Gly Gly Tyr Lys Glu Pro Ala Ala Pro
            180                 185                 190

Val Ser Ile Gln Arg Ser Ala Pro Gly Gly Gly Gly Lys Arg Tyr Arg
        195                 200                 205

Ala Val Tyr Asp Tyr Ser Ala Ala Asp Glu Asp Glu Val Ser Phe Gln
    210                 215                 220

Asp Gly Asp Thr Ile Val Asn Val Gln Gln Ile Asp Asp Gly Trp Met
225                 230                 235                 240

Tyr Gly Thr Val Glu Arg Thr Gly Asp Thr Gly Met Leu Pro Ala Asn
                245                 250                 255

Tyr Val Glu Ala Ile
            260

<210> 2
<211> 10
<212> PRT
<213> Homo sapiens

<400> 2
Gln Gln Ser Glu Leu Gln Ser Gln Val Arg
1               5                   10

<210> 3
<211> 9
<212> PRT
<213> Homo sapiens

<400> 3
Ala Cys Phe His Cys Glu Thr Cys Lys
1               5

<210> 4
<211> 10
<212> PRT
<213> Homo sapiens

<400> 4
Lys Pro Tyr Cys Asn Ala His Tyr Pro Lys
1               5                   10

<210> 5
<211> 10
<212> PRT
<213> Homo sapiens

<400> 5
Val Asn Cys Leu Asp Lys Phe Trp His Lys
1               5                   10

<210> 6
<211> 13
<212> PRT
<213> Homo sapiens

<400> 6
Gly Phe Ser Val Val Ala Asp Thr Pro Glu Leu Gln Arg
1               5                   10

<210> 7
<211> 12
<212> PRT
<213> Homo sapiens

<400> 7
Leu Lys Gln Gln Ser Glu Leu Gln Ser Gln Val Arg
1               5                   10

<210> 8
<211> 14
<212> PRT
<213> Homo sapiens

<400> 8
Met Gly Pro Ser Gly Gly Glu Gly Met Glu Pro Glu Arg Arg
1               5                   10

<210> 9
<211> 15
<212> PRT
<213> Homo sapiens

<400> 9
Thr Gly Asp Thr Gly Met Leu Pro Ala Asn Tyr Val Glu Ala Ile
1               5                   10                  15

<210> 10
<211> 15
<212> PRT
<213> Homo sapiens

<400> 10
Gly Lys Gly Phe Ser Val Val Ala Asp Thr Pro Glu Leu Gln Arg
1               5                   10                  15

<210> 11
<211> 14
<212> PRT
<213> Homo sapiens

<400> 11
Gln Ser Phe Thr Met Val Ala Asp Thr Pro Glu Asn Leu Arg
1               5                   10